# EUROPEAN PATENT APPLICATION

(11) **EP 3 421 494 A1**
(43) Date of publication of application: **02.01.2019**
(21) Application number: 17305825.6
(22) Date of filing: 29.06.2017
(51) Int. Cl.: C07K 16/28, A61P 35/00

(54) **USE OF ISATUXIMAB IN COMBINATION WITH AN ANTI-PD-1 ANTIBODY**

(71) Applicant: SANOFI, 75008 Paris (FR)
(72) Inventor: The designation of the inventor has not yet been filed
(74) Representative: Ribard, Carin Marie Lisa

(57) **Abstract**

Provided herein are combinations of isatuximab with an anti-PD1 for use in a method to treat relapsed and refractory multiple myeloma (RRMM). Provided herein are methods for treatment of MM comprising administering to the patient a safe and effective dose of isatuximab in combination with an anti-PD1 antibody, wherein the patient has previously received at least two prior lines of therapy, wherein one of the prior lines of therapy is a proteasome inhibitor and one of the prior lines of therapy is an immunomodulatory agent.

SEQUENCE LISTING

<110>  SANOFI

<120>  USE OF ISATUXIMAB IN COMBINATION WITH AN ANTI-PD-1 ANTIBODY

<130>  US2017/017

<160>  9

<170>  PatentIn version 3.5

<210>  1
<211>  444
<212>  PRT
<213>  Artificial Sequence

<220>
<223>  mAb heavy chain

<400>  1

Glu Val Gln Leu Leu Glu Ser Gly Gly Val Leu Val Gln Pro Gly Gly
1               5                   10                  15

Ser Leu Arg Leu Ser Cys Ala Ala Ser Gly Phe Thr Phe Ser Asn Phe
            20                  25                  30

Gly Met Thr Trp Val Arg Gln Ala Pro Gly Lys Gly Leu Glu Trp Val
        35                  40                  45

Ser Gly Ile Ser Gly Gly Gly Arg Asp Thr Tyr Phe Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Lys Gly Glu Asp Thr Ala Val Tyr Tyr Cys
                85                  90                  95

Val Lys Trp Gly Asn Ile Tyr Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser

165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Glu Ser Lys Tyr Gly Pro Pro Cys Pro
    210                 215                 220

Pro Cys Pro Ala Pro Glu Phe Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser Gln Glu Asp Pro Glu Val Gln Phe
            260                 265                 270

Asn Trp Tyr Val Asp Gly Val Glu Val His Asn Ala Lys Thr Lys Pro
        275                 280                 285

Arg Glu Glu Gln Phe Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Gly Leu Pro Ser Ser Ile Glu Lys Thr Ile Ser Lys Ala
                325                 330                 335

Lys Gly Gln Pro Arg Glu Pro Gln Val Tyr Thr Leu Pro Pro Ser Gln
            340                 345                 350

Glu Glu Met Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Arg Leu Thr Val Asp Lys Ser Arg Trp Gln Glu
                405                 410                 415

Gly Asn Val Phe Ser Cys Ser Val Met His Glu Ala Leu His Asn His
            420                 425                 430

Tyr Thr Gln Lys Ser Leu Ser Leu Ser Leu Gly Lys
        435                 440

<210>  2
<211>  117
<212>  PRT
<213>  Artificial Sequence

<220>
<223>  mAb heavy chain variable region

<400>  2

<210>  3
<211>  8
<212>  PRT
<213>  Artificial Sequence

<220>
<223>  hCDR1

<400>  3

Gly Phe Thr Phe Ser Asn Phe Gly
1               5

<210>  4
<211>  8
<212>  PRT
<213>  Artificial Sequence

<220>
<223>  hCDR2

<400>  4

Ile Ser Gly Gly Gly Arg Asp Thr
1               5


<210>  5
<211>  10
<212>  PRT
<213>  Artificial Sequence

<220>
<223>  hCDR3

<400>  5

Val Lys Trp Gly Asn Ile Tyr Phe Asp Tyr
1               5                   10


<210>  6
<211>  214
<212>  PRT
<213>  Artificial Sequence

<220>
<223>  mAb light chain

<400>  6

Asp Ile Gln Met Thr Gln Ser Pro Ser Ser Leu Ser Ala Ser Val Gly
1               5                   10                  15

Asp Ser Ile Thr Ile Thr Cys Arg Ala Ser Leu Ser Ile Asn Thr Phe
            20                  25                  30

Leu Asn Trp Tyr Gln Gln Lys Pro Gly Lys Ala Pro Asn Leu Leu Ile
        35                  40                  45

Tyr Ala Ala Ser Ser Leu His Gly Gly Val Pro Ser Arg Phe Ser Gly
    50                  55                  60

Ser Gly Ser Gly Thr Asp Phe Thr Leu Thr Ile Arg Thr Leu Gln Pro
65                  70                  75                  80

Glu Asp Phe Ala Thr Tyr Tyr Cys Gln Gln Ser Ser Asn Thr Pro Phe
                85                  90                  95

Thr Phe Gly Pro Gly Thr Val Val Asp Phe Arg Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  7
<211>  107
<212>  PRT
<213>  Artificial Sequence

<220>
<223>  mAb light chain variable region

<400>  7

<210>  8
<211>  6
<212>  PRT
<213>  Artificial Sequence

<220>
<223>  lCDR1

<400>  8

Leu Ser Ile Asn Thr Phe
1               5

<210>  9
<211>  9
<212>  PRT
<213>  Artificial Sequence

<220>
<223>  lCDR3

<400>  9

Gln Gln Ser Ser Asn Thr Pro Phe Thr
1               5

## Description

### BACKGROUND

Multiple myeloma (MM) is a malignant plasma cell disease that is characterized by clonal proliferation of plasma cells in the bone marrow and the production of excessive amounts of a monoclonal immunoglobulin (usually of the IgG or IgA type or free light chain [par protein, M protein or M component]). It is a disease predominantly associated with advancing age with more than 80% of patients aged 60 years or older. Patients under the age of approximately 65, that have symptomatic active disease and who are in good physical health will generally receive initial therapy with autologous stem cell transplantation (ASCT). To reduce the number of tumor cells (cytoreduction) before collecting stem cells, induction chemotherapy is administered. Induction treatment regimens include alkylating agents, dexamethasone alone, thalidomide plus dexamethasone, and vincristine, doxorubicin, and dexamethasone; however, the later 2 regimens are associated with higher toxicity (Terpos et al., Expert Opin Pharmacother. 2005;6(7):1127-42). Newer treatments with bortezomib alone, bortezomib combinations, and lenalidomide plus dexamethasone show some promise as induction therapy, and these agents demonstrate higher response rates and lower toxicity (Tepos et al., Palumbo et al.). In addition to these new treatments, daratumumab has been recently approved in US and Europe in single agent based on response rate of 29.2% (95%CI: 20.8-38.9) in late stage relapsed and refractory multiple myeloma (RRMM) patients previously treated with immunomodulatory agents and proteasome inhibitors (Lonial at al., J Clin Oncol 33, 2015 (suppl; abstr LBA8512)).

The current aim of MM therapy is to control the disease as effectively as possible, to maximize quality of life and to prolong survival. Treatments for relapsed and/or refractory disease are often referred to as salvage therapy. The initial chemotherapy regimen (e.g., bortezomib plus dexamethasone or bortezomib thalidomide plus dexamethasone or lenalidomide plus dexamethasone, or bortezomib plus melphalan plus prednisone depending if the patient was eligible for stem cell transplantation or not) can be reinstituted for relapsed/refractory disease if the disease relapsed more than 6 months after the last therapy ended. Subsequent treatment decisions are based on whether the patient experiences an indolent or aggressive relapse. In general, MM patients will receive an average of 4 to 8 different regimens during their lifespan utilizing agents such as proteasome inhibitors (e.g., bortezomib, ixazomib, or carfilzomib) and immunomodulatory agents (e.g., thalidomide, lenalidomide, or pomalidomide), monoclonal antibodies (elotuzumab), histone deacetylase (HDAC) inhibitors (panobinostat) alone or in combination. However, once a patient becomes refractory to those agents, survival is limited and newer treatment options are needed to treat patients after they have failed stem cell transplant (SCT), chemotherapy, proteasome inhibitors, and immunomodulatory agents.

Studies have shown the expression of PD-L1 on MM cells, and T and NK cells within the MM microenviroment expression PD-1(Görgün et al., Clin Cancer Res. 2015;15(20):4607-18). However, as a single agent, the anti-PD-1 antibody, nivolumab, had minimal activity in RRMM; tumor regression responses were not seen in MM patients except for one CR that occurred after the patient received radiation therapy (Lesokhin et al., J Clin Oncol. 2016;34(23):2698-2706). Miguel et al., Blood. 2015;126:505 and Shah et al., Clin Oncol 2016; 34, suppl; abstr describe the use of pembrolizumab in combination with dexamethasone and with either lenalidomide or pomalidomide, respectively, in RRMM. Both lenalidomide and pomalidomide have mandatory Risk Evaluation and Mitigation Strategies in place with the US Food and Drug Administration and are labeled with a "black box" warning due to embryo-fetal toxicity and venous and arterial thromoembolism.

Despite the improvement in patient outcomes with newer therapies, MM remains an incurable disease. Thus, safe and effective treatment of patients who have received at least two prior lines of therapy including a proteasome inhibitor and an immunomodulatory agent or who are double refractory to a proteasome inhibitor and an immunomodulatory agent remains an unmet medical need.

### SUMMARY

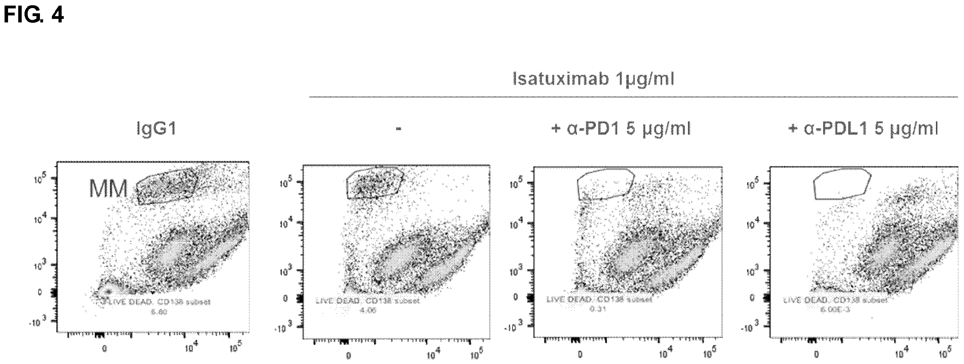

The inventors of have found that treatment of co-cultures of MM cells and PBMCs from healthy donors with isatuximab led to a partial killing of the myeloma cells, while addition of anti-PD-1 or anti-PD-L1 antibodies allowed fully eliminating multiple myeloma cells (see e.g. Example 1 and FIG. 4).

Therefore, provided herein are combinations of isatuximab with an anti-PD1 or an anti-PD-L1 antibody for use in a method to treat cancer, e.g. to treat a multiple myeloma such as relapsed and refractory multiple myeloma (RRMM). Provided herein are methods for treatment of MM comprising administering to the patient a safe and effective dose of isatuximab in combination with an anti-PD1 antibody, wherein the patient has previously received at least two prior lines of therapy, wherein one of the prior lines of therapy is a proteasome inhibitor and one of the prior lines of therapy is an immunomodulatory agent. The invention also pertains to isatuximab for use in combination with an anti-PD1 antibody or an anti-PD-L1 antibody for the treatment of cancer, e.g. multiple myeloma. Isatuximab can be administered to a patient having previously received at least two prior lines of therapy. One of the prior lines of therapy might be a proteasome inhibitor and one of the prior lines of therapy an immunomodulatory agent.

In some embodiments of the methods and uses provided herein, a safe and effective dose of isatuximab in combination with an anti-PD1 antibody comprises 10 mg/kg of isatuximab administered once every week for one 28 day cycle followed by 10 mg/kg every other week, and 1-3 mg/kg of anti-PD1 antibody administered every other week. In some embodiments of the methods and uses provided herein, a safe and effective dose isatuximab in combination with an anti-PD1 antibody comprises 10 mg/kg of isatuximab administered once every week for one 28 day cycle followed by 10 mg/kg every other week, and 1 mg/kg of anti-PD1 antibody administered every other week. In some embodiments of the methods and uses provided herein, a safe and effective dose of isatuximab in combination with an anti-PD1 antibody comprises 10 mg/kg of isatuximab administered every other week and 1 mg/kg of anti-PD1 antibody administered every other week.

In some embodiments of the methods and uses provided herein, the patient has received at least two months of treatment with the proteasome inhibitor and two months of treatment with the immunomodulatory agent prior to administering the isatuximab and the anti-PD1 antibody. In some embodiments, the patient has received at least two cycles of treatment with the proteasome inhibitor and two cycles of treatment with the immunomodulatory agent prior to administering the isatuximab and the anti-PD1 antibody. In some embodiments, a cycle of treatment comprises 28 days.

In some embodiments of the methods and uses provided herein, the patient has received three or more prior lines of therapy.

In some embodiments of the methods and uses provided herein, the patient has previously received another anti-CD38 antibody (i.e. an antibody that is not identical to isatuximab). In some embodiments, the most recent prior therapy received by the patient is another anti-CD38 antibody. In some embodiments, the other anti-CD38 antibody was daratumumab. In some embodiments, the other anti-CD38 antibody was MOR202. In some embodiments the other anti-CD38 antibody was TAK079.

In some embodiments of the methods and uses provided herein, the patient has MM that is refractory to one more prior lines of treatment. In some embodiments, the patient has MM that is refractory to one or more proteasome inhibitors. In another embodiment, the patient has MM that is refractory to one or more immunomodulatory agents. In another embodiment, the patient has MM that is refractory to a daratumumab. In some embodiments, the patient has MM that is refractory to one or more proteasome inhibitors and to one or more immunomodulatory agents. MM that is refractory to one or more proteasome inhibitors and one or more immunomodulatory agents is also referred to as double refractory.

In some embodiments of the methods and uses provided herein, the patient has relapsed MM. In some embodiments of the methods and uses provided herein, the patient has relapsed after one more prior lines of treatment. In some embodiments, the patient has relapsed after treatment with one or more proteasome inhibitors. In another embodiment, the patient has relapsed after treatment with one or more immunomodulatory agents. In another embodiment, the patient has relapsed after treatment with and anti-CD38 antibody such as daratumumab. In some embodiments, the patient has relapsed after treatment with one or more proteasome inhibitors and one or more immunomodulatory agents.

In some embodiments of the methods and uses provided herein, no other anti-myeloma drug or therapy, such as radiation therapy, is administered in combination with the isatuximab and anti-PD1 antibody. In some embodiments, dexamethasone is not administered in combination with the isatuximab and/or the anti-PD1 antibody.

In some embodiments of the methods and uses provided herein, isatuximab and the anti-PD1 antibody are administered simultaneously. In some embodiments, isatuximab and the anti-PD1 antibody are administered sequentially. In some embodiments, isatuximab is administered before the anti-PD1 antibody. In some embodiments, the anti-PD1 antibody is administered before isatuximab.

In some embodiments of the methods and uses provided herein, the anti-PD1 antibody is REGN2810.

In some embodiments of the methods and uses provided herein, the patient has at least one high risk cytogenic abnormality selected from the group consisting of del(17), t(4:14), and t(14:16).

Other objects, features and advantages will become apparent from the following detailed description. The detailed description and specific examples are given for illustration only since various changes and modifications within the spirit and scope of the invention will become apparent to those skilled in the art from this detailed description. Further, the examples demonstrate the principle of the invention and cannot be expected to specifically illustrate the application of this invention to all the examples where it will be obviously useful to those skilled in the prior art.

### BRIEF DESCRIPTION OF THE DRAWINGS

FIG. 1 shows the percentage of lysis of PD-L1 negative/positive RPMI-8226.CD38 MM cells by PD-1 negative/positive NK-92 human NK cells.
FIG. 2 shows the percentage of lysis of RPMI-8226.CD38 MM cells by NK-92.PD-1 in the presence of anti-PD-1 nivolumab (left) or anti-PD-L1 MPDL328A (right) antibodies.
FIG. 3 shows the percentage of CD14+ or CD56+ PBMCs that express PD-L1 or PD-1, respectively, after 6 days in culture alone or in the presence of U266 MM cells.
FIG. 4 shows isatuximab anti-MM myeloma activity alone and in combination with anti-PD-1 or anti-PD-L1 antibodies measured by flow cytometry.
FIG. 5 shows the heavy and light chain amino acid sequences of the anti-PD1 antibody REGN2810 (SEQ ID NOs: 1 and 6, respectively); the VH and VL sequences (SEQ ID NOs: 2 and 7, respectively, shown in italics), the three hCDRs (SEQ ID NOs: 2, 4, and 5) and the three ICDRs (SEQ ID NO:8, AAS, and SEQ ID NO:9).

### DETAILED DESCRIPTION

### INVESTIGATIONAL MEDICINAL PRODUCT

Isatuximab is a monoclonal antibody (mAb) that binds selectively to a unique epitope on the human surface antigen CD38. Isatuximab kills tumor cells via multiple biological mechanisms; antibody-dependent cellular-mediated cytotoxicity (ADCC), antibody-dependent cellular-mediated phagocytosis (ADCP), complement-dependent cytotoxicity (CDC) and direct induction of apoptosis (pro-apoptosis) without crosslinking. Isatuximab treatment of CD38 expressing cells also results in inhibition of CD38 enzymatic activity (Deckert et al., Clin Cancer Res. 2014 Sep 1;20(17):4574-83).

Programmed Death-1 protein (PD-1) functions as an 'immune checkpoint' in mediating peripheral T-cell tolerance and in avoiding autoimmunity. PD-1 binds to PD-L1 or PD-L2 and inhibits T-cell activation. (Pardoll 2012, Nature 12: 252-264).

In some embodiments of the methods and uses provided herein, the anti-PD-1 antibody can be a chimeric, humanized or human antibody. In some embodiments, the isolated human antibody is produced in a non-human animal model that has been engineered to express human immunoglobulin genes. In some embodiments, the anti-PD-1 antibody is isolated and/or recombinant. Examples of anti-PD-1 antibodies include REGN2810, nivolumab, pembrolizumab, MEDI0608 (formerly AMP-514; see, e.g., WO 2012/145493 and U.S. Patent No. 9,205,148), PDR001 (see, e.g., WO 2015/112900), PF-06801591 (see, e.g., WO 2016/092419) and BGB-A317 (see, e.g., WO 2015/035606).

In some embodiments, the anti-PD-1 antibody is one of those disclosed in WO 2015/112800 (such as those referred to as H1M7789N, H1M7799N, H1M7800N, H2M7780N, H2M7788N, H2M7790N, H2M7791N, H2M7794N, H2M7795N, H2M7796N, H2M7798N, H4H9019P, H4xH9034P2, H4xH9035P2, H4xH9037P2, H4xH9045P2, H4xH9048P2, H4H9057P2, H4H9068P2, H4xH9119P2, H4xH9120P2, H4xH9128P2, H4xH9135P2, H4xH9145P2, H4xH8992P, H4xH8999P and H4xH9008P in Table 1 of the PCT publication, and those referred to as H4H7798N, H4H7795N2, H4H9008P and H4H9048P2 in Table 3 of the PCT publication).

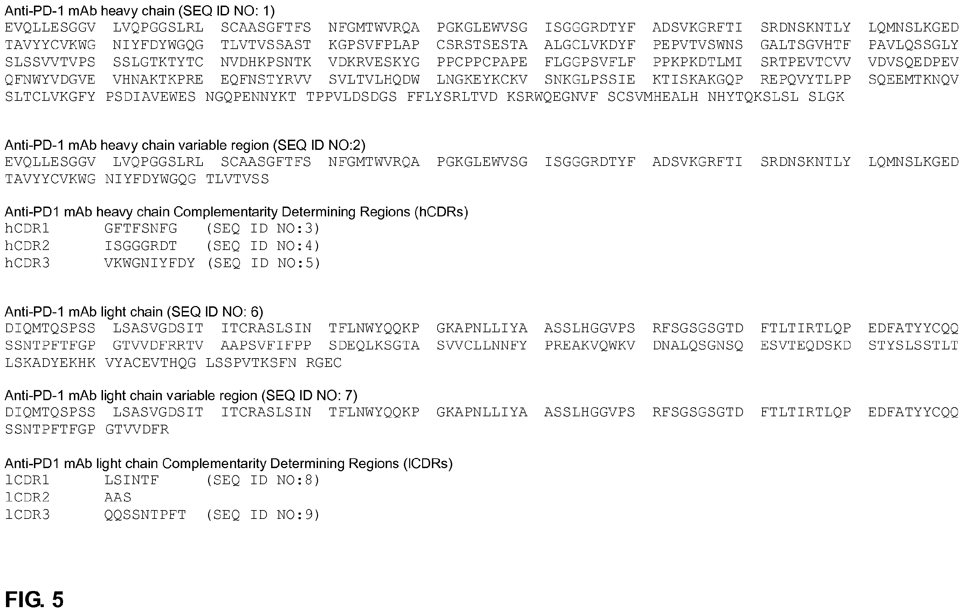

In some embodiments, the anti-PD-1 antibody comprises (i) the heavy and light chain amino acid sequences shown in FIG. 5 as SEQ ID NOs: 1 and 6, respectively; (ii) the VH and VL sequences in SEQ ID NOs: 2 and 7, and/or (iii) the three heavy chain CDRs in SEQ ID NOs:3-4 and the three light chain CDRs in SEQ ID NO: 8, the amino acid sequence AAS, and SEQ ID NO:9, respectively. An exemplary antibody comprising a heavy chain comprising the amino acid sequence of SEQ ID NO: 1 and a light chain comprising the amino acid sequence of SEQ ID NO: 6 is the fully human anti-PD-1 antibody known as REGN2810.

### FORMULATION

In some embodiments of the methods and uses provided herein, the isatuximab and anti-PD1 antibody are formulated as a pharmaceutical composition suitable for parenteral administration. Suitable formulations typically comprise the active ingredient combined with a pharmaceutically acceptable carrier, such as sterile water or sterile isotonic saline. Such formulations may be prepared, packaged, or sold in a form suitable for bolus administration, continuous administration, or for dilution prior to i.v. administration, for example. Injectable formulations may be prepared, packaged, or sold in unit dosage form, such as in ampoules or in multi dose containers containing a preservative. Suitable parenteral formulations can also comprise aqueous solutions which may contain excipients such as salts, carbohydrates and buffering agents (at a pH of from 3 to 9, for example).

### INDICATIONS

As used herein, refractory myeloma is defined as disease that is non-responsive (failure to achieve minimal response or develops progressive disease (PD) while on therapy) while on primary or salvage therapy, or progresses within 60 days of last therapy. There are two categories of refractory myeloma. Relapsed and refractory myeloma (RRMM): RRMM is defined as disease that is non-responsive while on salvage therapy or progresses within 60 days of last therapy in patients who have achieved minimal response (MR) or better at some point previous and then experiencing disease progression. Primary refractory myeloma: primary refractory myeloma is defined as disease that is non-responsive in patients who have never achieved MR or better with any therapy. Primary refractory myeloma includes patients who never achieve MR or better in whom there is no significant change in M protein and no evidence of clinical progression; as well as primary refractory, progressive disease where patients meet criteria for true progressive disease.

As used herein relapsed myeloma is defined as previously treated myeloma which progresses and requires the initiation of salvage therapy but does not meet the criteria for either primary refractory myeloma or RRMM. (Adapted from Rajkumar VS, Harousseau J-Let al Consensus recommendations for the uniform reporting of clinical trials: report of the International Myeloma Workshop Consensus Panel 1. Blood 2011; 117(18):4691-95).

### PRIOR LINES OF THERAPY

As used herein, a line of therapy greater than or equal to 1 complete cycle of a single agent, a regimen of a combination of several drugs, or a planned sequential therapy of various regimens (e.g., 3-6 cycles of initial therapy with bortezomib-dexamethasone followed by stem cell transplantation consolidation, and lenalidomide maintenance is considered 1 line).

New line of Therapy: As used herein a given myeloma treatment is considered a new line of therapy if any one of the following three conditions are met. 1) Start of a new line of treatment after discontinuation of a previous line. If a treatment regimen is discontinued for any reason and a different regimen is started, it is considered a new line of therapy. A regimen is considered to have been discontinued if all the drugs in that given regimen have been stopped. A regimen is not considered to have been discontinued if some of the drugs of the regimen, but not all, have been discontinued. 2) The unplanned addition or substitution of 1 or more drugs in an existing regimen. Unplanned addition of a new drug or switching to a different drug (or combination of drugs) due to any reason is considered a new line of therapy. 3) Stem cell transplantation (SCT): In patients undergoing more than one round of SCT, except in the case of a planned tandem SCT with a predefined interval (such as 3 months), each SCT (autologous or allogeneic) is considered a new line of therapy regardless of whether the conditioning regimen used is the same or different. Planned tandem SCT is considered 1 line. Planned induction and/or consolidation, maintenance with any SCT (frontline, relapse, autologous or allogeneic) is considered 1 line.

The reasons for discontinuation, addition, substitution, or SCT do not influence how lines are counted. It is recognized that reasons for change may include end of planned therapy, toxicity, progression, lack of response, inadequate response.

If a regimen is interrupted or discontinued for any reason and the same drug or combination is restarted without any other intervening regimen, then it is counted as a single line. If a regimen is interrupted or discontinued for any reason, and then restarted at a later time point but 1 or more other regimens were administered in between, or the regimen is modified through the addition of 1 or more agents, then it is counted as 2 lines.

Modification of the dosing of the same regimen should not be considered a new line of therapy. Based on Rajkumar, Richardson and San Miguel. Guidelines for the determination of the number of prior lines of therapy in multiple myeloma Blood 2015;126[7]:921-922.

### RESPONSE CRITERIA

In some embodiments of the methods and uses provided herein, a safe and effective dose of isatuximab in combination with an anti-PD1 antibody results in an Overall Response Rate of at least 43% at a dose and administration regimen wherein no more than 1 out of 6 patients treated at the selected dose regimen experience dose limiting toxicity during one 28 day cycle of therapy. In other embodiments, the ORR is at least 44, 45, 46, 47, 48, 49, or 50% at a dose and administration regimen wherein no more than 1 out of 6 patients treated at the selected dose regimen experience dose limiting toxicity during one 28 day cycle of therapy. In some embodiments, the safe and effective dosage regimen comprises administering isatuximab at 10 mg/kg every week for 4 weeks followed by 10 mg/kg every other week and administering the anti-PD1 antibody at 3 mg/kg every other week. In some embodiments, the safe and effective dosage regimen comprises administering isatuximab at 10 mg/kg every week for 4 weeks followed by 10 mg/kg every other week and administering the anti-PD1 antibody at 1 mg/kg every other week. In some embodiments, the safe and effective dosage regimen comprises administering isatuximab at 10 mg/kg every other week and administering the anti-PD1 antibody at 1 mg/kg every other week.

### IMWG Response Criteria

Disease response is assessed using the updated International Myeloma Working Group Response Criteria (IMWG). A confirmation assessment for disease response within 4 weeks is required in this protocol (either PR or better, or PD). Paraprotein value on Cycle 1 Day 1 is taken as baseline value for response assessment.

### Adapted from updated International Myeloma Working Group Response Criteria

| **IMWG MRD criteria (requires a complete response as defined below)** | |
|---|---|
| Sustained MRD-negative | MRD negativity in the marrow (NGF or NGS, or both) and by imaging as defined below, confirmed minimum of 1 year apart. Subsequent evaluations can be used to further specify the duration of negativity (eg, MRD-negative at 5 years) |
| Flow MRD-negative | Absence of phenotypically aberrant clonal plasma cells by NGF on bone marrow aspirates using the EuroFlow standard operation procedure for MRD detection in multiple myeloma (or validated equivalent method) with a minimum sensitivity of 1 in 10⁵ nucleated cells or higher |
| Sequencing MRD-negative | Absence of clonal plasma cells by NGS on bone marrow aspirate in which presence of a clone is defined as less than two identical sequencing reads obtained after DNA sequencing of bone marrow aspirates using the LymphoSIGHT platform (or validated equivalent method) with a minimum sensitivity of 1 in 10⁵ nucleated cells or higher |
| Imaging-positive MRD-negative | MRD negativity as defined by NGF or NGS plus disappearance of every area of increased tracer uptake found at baseline or a preceding PET/CT or decrease to less mediastinal blood pool SUV or decrease to less than that of surrounding normal tissue |

| **Standard IMWG response criteria** | |
|---|---|
| **Response** | **IMWG criteria** |
| CR | • Negative immunofixation on the serum and urine and |
| | • disappearance of any soft tissue plasmacytomas and |
| | • <5% plasma cells in bone marrow aspirates. |
| | • A normal FLC ratio of 0.26-1.65 is required. |
| | **Two consecutive assessments are needed. No known evidence of progressive disease or new bone marrow lesions if radiographic studies were performed** |
| sCR | CR as defined above plus: |
| | • normal FLC ratio (0.26 to 1.65) and |
| | • absence of clonal cells in bone marrow by immunohistochemistry (κ/λ ratio ≤4:1 or ≥1:2 for κ and λ patients, respectively, after counting ≥100 plasma cells) |
| | **Two consecutive assessments of laboratory parameters are needed. No known evidence of progressive disease or new bone marrow lesions if radiographic studies were performed** |
| VGPR | • Serum and urine M-protein detectable by immunofixation but not on electrophoresis or |
| | • ≥90% reduction in serum M-protein plus urine M-protein level <100 mg/24 h. |
| | • FLC only: a ≥90% decrease in the difference between involved and uninvolved FLC levels. |
| | **Two consecutive assessments are needed. No known evidence of progressive disease or new bone marrow lesions if radiographic studies were performed** |
| PR | • ≥50% reduction of serum M-protein and reduction in 24 hours urinary M-protein by ≥90% or to <200 mg/24 h |
| | • If serum and urine M-protein are unmeasurable, a ≥50% decrease in the difference between involved and uninvolved FLC levels is required in place of the M-protein criteria |
| | • If serum and urine M-protein are unmeasurable, and serum-free light assay is also unmeasurable, ≥50% reduction in plasma cells is required in place of M-protein, provided baseline bone marrow plasma-cell percentage was >30%. In addition to the above listed criteria, if present at baseline, a ≥50% reduction in the size (SPD) of soft tissue plasmacytomas is also required |
| | **Two consecutive assessments are needed. No known evidence of progressive disease or new bone marrow lesions if radiographic studies were performed** |
| MR | ≥25% but ≤ 49% reduction in serum M-protein and reduction in 24h urine M-protein by 50-89%, which still exceed 200 mg/24H. |
| | In addition to the above listed criteria, if present at baseline, ≥50% reduction in size (SPD) of soft tissue plasmacytomas is also required |
| | **No known evidence of progressive disease or new bone marrow lesions if radiographic studies were performed** |
| Stable Disease | • Not meeting criteria for CR, VGPR, PR, MR or progressive disease |
| | **Two consecutive assessments are needed. No known evidence of progressive disease or new bone marrow lesions if radiographic studies were performed** |
| Progressive disease | Any one or more of the following criteria: |
| | Increase of ≥25% from lowest confirmed value in any one of the following criteria: |
| | • Serum M-protein (the absolute increase must be ≥0.5 g/dL) |
| | • Serum M-protein increase ≥1 g/dL if the lowest M component was ≥5 g/dL |
| | • Urine M-component (the absolute increase must be ≥200 mg/24 h) |
| | • In patients without measurable serum and urine M-protein levels, the difference between involved and uninvolved FLC levels (absolute increase must be >10 mg/dL) |
| | • In patients without measurable serum and urine M-protein levels and without measurable involved FLC levels, bone marrow plasma-cell percentage irrespective of baseline status (absolute increase must be ≥10%) |
| | • Appearance of new lesion(s), ≥50% increase from nadir in SPD of >1 lesion, or ≥50% increase in the longest diameter of a previous lesion >1 cm in short axis; |
| | ≥50% increase in circulating plasma cells (minimum of 200 cells per µL) if this is the only measure of disease |
| | **Two consecutive assessments are needed. No known evidence of progressive disease or new bone marrow lesions if radiographic studies were performed** |

| | |
|---|---|
| Abbreviations: CR, complete response; FLC, free light chain; IMWG, International Myeloma Working Group; M, monoclonal; MRD, minimal residual disease; NGF, next-generation flow; NGS, next-generation sequencing; PD, progressive disease; PR, partial response; sCR, stringent complete response; SD, stable disease; SPD, sum of the products of the maximal perpendicular diameters of measured lesions; SUV, maximum standardized uptake value; VGPR, very good partial response. | |

Definitions of Response and Progression are based on IMWG Uniform Reporting Criteria:
- Any response (sCR, CR, VGPR, PR) or progression is confirmed by two consecutive disease assessments. A disease assessment at one time point not matched by the same disease assessment at the next time point is considered unconfirmed (except for progression by imaging, bone marrow PC counts, where one time point is adequate for confirmed progression).
- Urine M-protein is not needed to document partial response or minor response if baseline urine M-protein was not measurable; however, it is still required for complete response and very good partial response.
- Serum FLC levels should only be used for response assessment when both the serum and urine M-component levels are deemed not measurable.
- Documentation of response requires two consecutive readings of the applicable disease parameter (serum M-protein, urine M-protein), performed at any time (no minimum interval is required, it can be done the same day); however, to confirm response or progressive disease, two discrete samples are required; testing cannot be based upon the splitting of a single sample.
- Patients will continue in the last confirmed response category until there is confirmation of progression or improvement to a higher response status; patients cannot move to a lower response category.
- Percent decreases for response calculations are from baseline values (Cycle 1, Day 1).
- Percent increases for progression calculations are from lowest response values or baseline values, whichever is the smaller number. The lowest value does not need to be confirmed.
- The lowest value before suspected progression is used for calculation of progression; if a serum and/or urine spike is considered too low to quantitate, this value can be assigned as zero as a baseline for documentation of subsequent progressive disease. Patients will be considered to have progressive disease if they meet the criteria for progression by a variable that was not considered measurable at baseline; however, for patients who had a measurable serum or urine M-spike at baseline, progression cannot be defined by increases in serum FLC alone.

Examples have been set forth below for the purpose of illustration and to describe certain specific embodiments of the invention. However, the scope of the claims is not to be in any way limited by the examples set forth herein.

### Example 1 Pre-clinical Studies

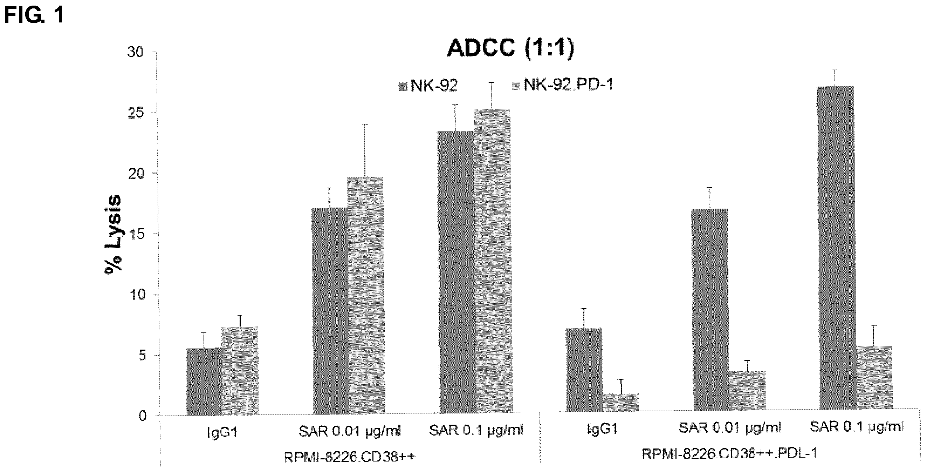

RPMI-8226 multiple myeloma cells were engineered to express PD-L1 and NK-92 human NK cells were engineered to express PD-1. As shown in FIG. 1, isatuximab treatment of target cells that do not express PD-L1 results in similar lysis by PD-1 negative (dark grey) or PD-1 positive NK-92 cells (light grey). Target cells expressing PD-L1 are efficiently lysed by PD-1 negative NK cells, but cannot be lysed by NK cells expressing PD-1. This data demonstrates that the PD-1/PD-L1 interaction can impair isatuximab induced ADCC.

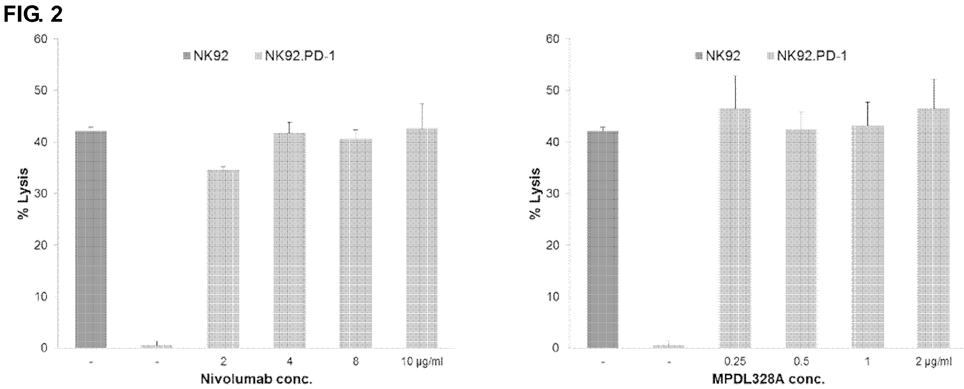

Addition of different concentrations of anti-PD-1 (nivolumab) or anti-PD-L1 (MPDL-3280A) antibodies, isatuximab ADCC activity was fully restored, as shown in FIG. 2.

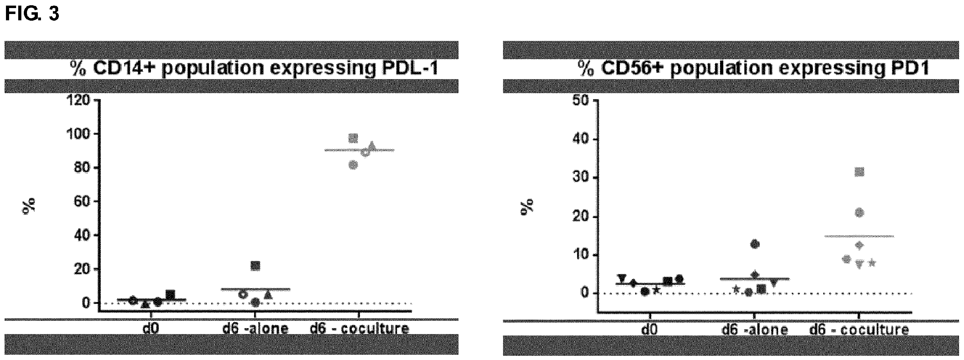

The isatuximab/anti-PD-1 or PD-L1 combination was also tested in a co-culture system. Upon a 6 day co-culture of CD38 overexpressing U266 MM cells and PBMCs isolated from healthy donors, PD-L1 expression is induced in about 100% of the monocytes and PD-1 expression is induced on NK cells, as shown in FIG. 3.

Treatment of the co-cultures with isatuximab at 1 µg/ml led to a partial killing of the myeloma cells. By addition of anti-PD-1 or anti-PD-L1 antibodies at 5 µg/ml, the MM cells were fully eliminated, as shown in FIG. 4.

### Example 2 Clinical Study

In this open-label Phase 1 / 2 clinical study of isatuximab in combination with REGN2810, the safety, tolerability, and efficacy of treatment of RRMM by administering isatuximab in combination with REGN2810 as described herein is evaluated.

### PRIMARY OBJECTIVE(S)

To determine the safety and tolerability of the combination of isatuximab and REGN2810.

To determine the overall response rate (ORR, CR+VGPR+PR) of the combination of isatuximab and REGN2810 in patients with RRMM based on International Myeloma Working Group (IMWG) criteria.

### SECONDARY OBJECTIVE(S)

To determine the following efficacy measurements:
Clinical benefit rate (CBR, CR + VGPR + PR + Minimal response [MR]),
Duration of response (DOR),
Time to response (TTR),
Progression free survival (PFS),
Overall survival (OS).
To determine the pharmacokinetic profile of isatuximab and REGN2810 when given in combination.
To assess the immunogenicity of isatuximab and REGN2810 when given in combination.

### EXPLORATORY OBJECTIVE(S)

To explore the minimal residual disease (MRD) in patients achieving a CR.

To assess the relationship between immune phenotypes, immune regulatory marker expression, adaptive immune response and parameters of clinical response.

To explore central/effector memory T cell proliferation.

The study is conducted in two parts. Isatuximab is administered at a dose of 10 mg/kg once a week for four weeks (QWx4) (one 28 d cycle) followed by once every other week (Q2W) 3 mg/kg of the anti-PD1 antibody REGN2810 is administered Q2W.

The study is conducted in two parts. Isatuximab is administered at a dose of 10 mg/kg once a week for four weeks (QWx4) (one 28 d cycle) followed by once every other week (Q2W) 3 mg/kg of the anti-PD1 antibody REGN2810 is administered Q2W. Isatuximab and REGN2810 are also referred to herein as "study treatments."

Dose de-escalation can be performed as defined in the Table below:

**Treatment dose and schedule**

| **Dose level** | **Isatuximab** | **REGN2810** |
|---|---|---|
| Starting dose (SD) | 10 mg/kg | 3 mg/kg |
| | QWx4 >Q2W | Q2W |
| Minus 1 (DL-1) | 10 mg/kg | 1 mg/kg |
| | QWx4 >Q2W | Q2W |
| Minus 2 (DL-2) | 10 mg/kg | 1 mg/kg |
| | Q2W | Q2W |

Patients receiving the starting dose will be evaluated for Dose Limiting Toxicity (DLT).

### Maximum administered dose / Maximum tolerated dose

The MAD is defined as the lowest dose at which at least 3 out of 12 patients (SD and DL-1) or at least 2 out of 6 patients (DL-2) experienced study treatment related DLTs during the initial DLT observation period (Cycle 1). The MTD is defined as the highest DL at which no more than 1 out of 6 patients or 2 out of 12 experienced a study treatment related DLT.

### PART 2

In the Phase 2 part, patients will be evaluated at the recommended phase 2 dose (RP2D) to further assess the safety and efficacy of the combination therapy. Study treatment can continue, until disease progression, unacceptable toxicity, or patient/investigator decision to continue. A cycle is 28 days, and deemed to have been delayed if the treatment is >3 days beyond the theoretical day of treatment. Patients will be followed until progression of disease (PD) or death for a maximum of 1 year after last patient is treated. This will allow the study to have a proper evaluation of the combination in term of PFS and OS.

### DOSE DELAYS/ MODIFICATIONS

Dose modifications are permitted according to the guidelines described in this section. Dose adjustment and/or cycle delay are permitted in case of toxicity. Dose adjustments will be made according the worst grade of toxicity observed within a cycle. If a patient experiences several toxicities and there are conflicting recommendations, the most conservative dose adjustment recommended should be followed. Once a dose has been decreased, intra-patient re-escalation back to the previous dose level is not permitted. Administration of the study treatment is discontinued in the event of a TEAE that persists despite appropriate dose modifications or any other AE that, in the opinion of the Investigator, warrants discontinuation.

If one of the 2 drugs (REGN2810 or isatuximab) is prematurely permanently discontinued, the other drug can be continued until disease progression, unacceptable adverse reaction, patient's refusal of further treatment, or in the absence of a clear benefit from therapy. The end of treatment assessment in this case will be 30 day after the date of the last IMP administration.

### Dose delay and dose omission

Within a cycle, the treatment window is ±1 day for each of the weekly administrations and ±2 days for each of the Q2W administrations. Within a cycle, a dose is deemed to have been delayed if the treatment is >1 day beyond the theoretical day of treatment for weekly dose, and >2 days beyond the theoretical day of treatment for Q2W dose. Patient will receive the next infusion after recovery of the toxicity as described herein. If infusion has been delayed by 2 days within a cycle, next study treatment is administered at the planned time interval between 2 administrations (e.g., Day 15 Cycle 1 administration is actually administered on Day 17, the planned Day 22 is administered on Day 24).

Patients may have dose omission if toxicity occurs and does not recover as follows:
In Cycle 1 if toxicity occurs and does not recover on the day of planned infusion or within the following 3 days, infusion of isatuximab and/or REGN2810 may be omitted.
In Cycle 2 and beyond, if toxicity occurs and does not recover on the day of planned infusion or within the following 7 days, infusion of isatuximab and/or REGN2810 may be omitted. In case of consecutive dose omissions for the recovery of toxicity, following rules should be followed for restart or discontinue the treatment:
   Dose interruption (cycle delay) up to 14 days, it is per Investigator's decision to restart the treatment of REGN2810 and isatuximab.
   After dose interruption of >14 days, it is per Investigator's decision to restart the treatment of REGN2810 and isatuximab, if a clear benefit from therapy is observed and after consultation with the sponsor. Treatment with isatuximab and REGN2810 must be definitely discontinued if the interruption is longer than 84 days.

### Dose modifications

Dose reduction of REGN2810 is permitted for patients treated at the starting dose level and will consist in time interval increase between 2 planned infusions.

### Dose reduction guidance

| | **Isatuximab*** | | **REGN2810** | |
|---|---|---|---|---|
| **Dose level** | **Dose (mg/kg)** | **Frequency** | **Dose (mg)** | **Frequency** |
| Starting dose | 10 | QW x4 >Q2W | 3 mg/kg | Q2W |
| Dose reduction | 10 | QW x4 >Q2W | 1 mg/kg | Q2W |

| | | | | |
|---|---|---|---|---|
| * No dose reduction for isatuximab | | | | |

Potential DLTs are defined as the occurrence of any of the following adverse reactions, unless clearly unrelated to the study therapies at first cycle:
Hematological abnormalities is defined as any of the following that is related to the study treatment:
   Grade 4 neutropenia lasting more than 7 consecutive days.
   Grade 3 to 4 neutropenia complicated by fever (temperature ≥38.5°C on more than one occasion) or microbiologically or radiographically documented infection.
      Grade 3 to 4 thrombocytopenia associated with bleeding that requiring clinical intervention. Platelet transfusions in the absence of bleeding will not be considered a DLT because thrombocytopenia is an anticipated complication of MM, particularly in a heavily pretreated patient population, and patients can enter the study with pre-existing thrombocytopenia.
      Non-hematologic DLT is defined as any of the followings that is related to the study treatments:
      Grade 4 non-hematologic AE.
      Grade ≥2 uveitis.
      Grade 3 non-hematological AE lasting >3 days despite optimal supportive care. Except:
         Grade 3 fatigue,
         Allergic reaction/hypersensitivity attributed to isatuximab or REGN2810,
         Laboratory abnormality that worsens to Grade 3 or 4 but is not clinically significant per investigator and study committee.
         Delay in initiation of the 2nd cycle >14 days due to treatment related laboratory abnormalities/ AE.

### INCLUSION CRITERIA

Patients must have a known diagnosis of multiple myeloma with evidence of measurable disease, as defined below:
- Serum M-protein ≥1 g/dL (≥0.5 g/dL in case of IgA disease),
AND/OR
- Urine M-protein ≥200 mg/24 hours,
OR
- In the absence of measurable M-protein, serum immunoglobulin free light chain ≥10 mg/dL, and abnormal serum immunoglobulin kappa lambda free light chain ratio (<0.26 or >1.65).

Patients must have received prior treatment with an immunomodulatory agent (for ≥2 cycles or ≥2 months of treatment) and a proteasome inhibitor (for ≥2 cycles or ≥2 months of treatment). Patients must have received EITHER
- at least 3 prior lines of therapy (Note: Induction therapy and stem cell transplant ± maintenance will be considered as one line).
OR
- Patients whose disease is double refractory to an immunomodulatory agent and a proteasome inhibitor (disease progression has occurred while on or within 60 days from last treatment).

Patient with refractory disease only, must have achieved MR or better with any anti-myeloma therapy (i.e., primary refractory disease is not eligible).

Patients must have a disease that is refractory to the most recent prior line therapy (disease progression has occurred while on treatment or within 60 days from last treatment).

### EXCLUSION CRITERIA

Has any concurrent hematology malignancy other than multiple myeloma, including:
- Active primary AL amyloidosis,
- Concomitant plasma cell leukemia.

Has prior exposure to:
- Isatuximab or participated clinical studies with isatuximab,
- Any agent (approved or investigational) that blocks the PD-1/PD-L1 pathway.

Diagnosed or treated for another malignancy within 3 years prior to the study treatment with the exception of resected/ablated basal or squamous cell carcinoma of the skin, or carcinoma in situ of the cervix, or other local tumors considered cured by local treatment, or low risk prostate cancer after curative therapy;
Evidence of other immune related disease /conditions, including:
- Ongoing or recent (within 2 years) evidence of significant autoimmune disease that required systemic immunosuppressive treatment. Replacement therapy (e.g., thyroxine, insulin, or physiologic corticosteroid replacement therapy for adrenal or pituitary insufficiency, etc.) is not considered a form of systemic treatment,
History of moderate immune-mediated acute drug reactions (e.g., colitis, hepatitis, etc.). History of non-infectious pneumonitis requiring steroids or current pneumonitis; history of the thoracic radiation.
Has received a live-virus vaccination within 30 days of planned treatment start. Seasonal flu vaccines that do not contain live virus are permitted.
Known to be HIV+, HBV+ or hepatitis A, B, or C active Infection.
Has allogenic HSC transplant.
Has prior exposure to treatment, including:
- Any anti-myeloma drug treatment including dexamethasone within 14 days before study entry (90 days if prior anti-CD38 treatment),
Any major procedure within 14 days before the initiation of the study treatment: plasmapheresis, major surgery (kyphoplasty is not considered major procedure), radiotherapy, Any investigational drugs within 28 days or 5 half-lives from the 1 st study treatment, whichever is longer.

Congestive heart failure (New York Heart Association class III to IV) myocardial infarction within 6 months or with reduced ejection fraction, symptomatic coronary artery disease, major clinically significant electrocardiogram (ECG) and echocardiogram abnormalities, significant ventricular arrhythmias.

Ongoing adverse events Grade ≥2 (excluding alopecia and those listed in eligibility criteria) from any prior anti-cancer therapy ≥(NCI-CTC v4.03).

Eastern Cooperative Oncology Group (ECOG) performance status (PS) >2.

Inadequate hematological function including:
- Platelets <50,000/mm3. Patient should be platelet transfusion independent for 2 weeks prior to screening lab values),
- ANC ≤1000/mm3 (1 x 109/L). (use of colony-stimulating factors to achieve these counts is allowed),
- Hemoglobin <8.0 g/dL (patients may receive red blood cell transfusion or receive supportive care such as erythropoietin and darbepoetin in accordance with institutional guidance).

Inadequate liver findings including:
- Total bilirubin >2xULN,
- AST and/or ALT >3xULN.

Inadequate renal function: Estimated glomerular filtration rate (eGFR) <30 mL/min/1.73m2 (MDRD Formula). See Appendix A for MDRD equation.

Corrected serum calcium >14 mg/dl (>3.5 mmol/l).

### DOSING SEQUENCE

All patients will receive isatuximab and REGN2810.

On Day 1 and 15 each cycle patients will receive:

### Pre-medications

- Acetaminophen 650 mg to 1000 mg PO (or equivalent).
- Ranitidine 50 mg IV (or equivalent: other approved H2 antagonists [e.g., cimetidine], oral proton pump inhibitors [e.g., omeprazole, esomeprazole]).
- Diphenhydramine 25-50 mg IV (or equivalent: e.g., cetirizine, promethazine, dexchlorpheniramine, according to local approval and availability. Intravenous route is preferred for at least the first 4 infusions).
- Methylprednisolone 100 mg IV (or equivalent)
followed by
REGN2810 infusion over 30 minutes,
followed by
Isatuximab iv infusion over approximately 2-4 hours.

In Cycle 1 Day 8 and 22, patients will receive:
Pre-medications as described above,
followed by
Isatuximab iv infusion over approximately 2-4 hours.

### RATE AND DURATION OF INFUSION

The duration of infusion for REGN2810 is 30 minutes.

For isatuximab, the rate of infusion for isatuximab should be initiated at 175 mg/hour:
First infusion: initiate infusion at 175 mg/hour. In the absence of IARs after 1 hour of infusion, increase infusion rate by 50 mg/hour increments every 30 minutes, to a maximum of 400 mg/hour.

Subsequent infusions: initiate infusion at 175 mg/hour. In the absence of IAR after 1 hour of infusion, increase rate by 100 mg/hour increments every 30 minutes, to a maximum of 400 mg/hour.

### EFFICACY

### Primary endpoint

The efficacy of the combination is assessed by ORR. ORR is defined as the proportion of patients with CR (including sCR), VGPR and PR as assessed by investigators using the IMWG response criteria.

The following disease assessment procedures will be performed at screening (for eligibility) and again at Cycle 1 Day 1 prior to study treatment administration (baseline for response assessment) and then Day 1 of every cycle during treatment up to progression and for patients who discontinue study treatment for reasons other than progression, every 4 weeks during follow-up until PD:
M-protein quantification (serum and urine)
Free Light Chain (FLC) quantification
Quantitative immunoglobulins
In addition, other examinations for disease assessment will be done as clinically indicated (e.g., to confirm a response, suspected PD) as below
   - Bone marrow aspiration,
   - Bone disease assessment,
   - Extramedullary disease (plasmacytoma) assessment (including bone plasmacytoma).

Response/progression will be determined according to IMWG criteria.
Response/progression based on M-protein and FLC will be confirmed based on 2 consecutive assessments.

Progressive disease is defined for patients with measurable disease with one of the following:
- Increase of ≥25% in serum M-protein from nadir (the absolute increase must be ≥0.5 g/dL) in 2 consecutive assessments; serum M-protein increases ≥1 g/dL in 2 consecutive assessments are sufficient to define relapse if starting M-protein t is ≥5 g/dL and/or,
- Increase of ≥25% in urine M-protein from nadir (the absolute increase must be ≥200 mg/24 h) in 2 consecutive assessments and/or,
- Only in subjects without measurable serum and urine M-protein levels: increase of ≥25% in the difference between involved and uninvolved FLC levels and absolute increase must be >10 mg/dL and/or,
- Definite development of new bone lesions or soft tissue extramedullary disease, or increase ≥50% from nadir in the sum of perpendicular diameters of existing soft tissue extramedullary disease lesions if >1 lesion, or ≥50% increase in the longest diameter of a previous soft tissue extramedullary disease lesion >1 cm in short axis.
Clinical deterioration will not be considered progression in the primary analysis of ORR.

Measurable disease is defined by at least one of the following measurement:
- Serum M-protein ≥1 g/dL or ≥0.5 g/dL for patients with IgA MM.
- Urine M-protein ≥200 mg/24h.
- In the absence of measurable M-protein, serum immunoglobulin FLC level ≥10 mg/dL provided abnormal serum immunoglobulin kappa lambda FLC ratio (<0.26 or >1.65).

### Secondary endpoints

Clinical Benefit Rate (CBR): defined as the proportion of patients with CR (including sCR), VGPR, PR and minimal response (MR) as assessed by investigators using the IMWG response criteria.

Duration of Response (DOR): defined as the time from the date of the first response (≥PR) that is subsequently confirmed to the date of first confirmed disease progression (PD) or death, whichever happens first. PD will be determined according to IMWG criteria. In the absence of the confirmation of subsequent disease progression or death before the analysis cut-off date or the date of initiation of a further anticancer treatment, the DOR will be censored at the date of the last valid disease assessment not showing disease progression performed prior to initiation of a further anticancer treatment and the analysis cut-off date, whichever is earlier. DOR will not be calculated for patients that do not achieve a PR or better.

Time to Response (TTR): defined as time from first study treatment administration (C1D1) to first response (PR or better) that is subsequently confirmed.

Progression Free Survival (PFS): defined as time from the first study treatment administration to the date of first documentation of progressive disease (PD) that is subsequently confirmed or the date of death from any cause. Same censoring rules as DOR will be used.

Overall Survival (OS): defined as the time from the first study treatment administration to death from any cause. Patients without death prior to the analysis cutoff date will be censored at the last date the patient was known to be alive or the cutoff date whichever comes first.

Urine M-protein is not needed to document partial response or minor response if baseline urine M-protein was not measurable; however, it is still required for complete response and very good partial response.

Serum FLC levels should only be used for response assessment when both the serum and urine M-component levels are deemed not measurable.

Documentation of response requires two consecutive readings of the applicable disease parameter (serum M-protein, urine M-protein), performed at any time (no minimum interval is required, it can be done the same day); however, to confirm response or progressive disease, two discrete samples are required; testing cannot be based upon the splitting of a single sample.

Patients will continue in the last confirmed response category until there is confirmation of progression or improvement to a higher response status; patients cannot move to a lower response category.

Percent decreases for response calculations are from baseline values (Cycle 1, Day 1).

Percent increases for progression calculations are from lowest response values or baseline values, whichever is the smaller number. The lowest value does not need to be confirmed.

The lowest value before suspected progression will be used for calculation of progression; if a serum and/or urine spike is considered too low to quantitate, this value can be assigned as zero as a baseline for documentation of subsequent progressive disease. Patients will be considered to have progressive disease if they meet the criteria for progression by a variable that was not considered measurable at baseline; however, for patients who had a measurable serum or urine M-spike at baseline, progression cannot be defined by increases in serum FLC alone.

### DURATION OF STUDY PARTICIPATION

The duration of the study for a patient will include a period for screening of up to 21 days. The cycle duration is 28 days. Patients will continue treatment until disease progression, unacceptable adverse events, consent withdrawal, or any other reason.

After study treatment discontinuation, patients will return to the study site 30 days (+5 days) after the last dose of study treatments, for end-of-treatment assessments, and 60 days after the last dose of study treatments for ADA assessment. If ADA test at Day 60 is positive or inconclusive, ADA testing will be repeated every 30 days until negative.

The post-treatment follow up period (including an extended safety follow-up period of 90 days after the last dose of study treatment) is defined as the time period from 31 days after the last dose of study treatments until death or study cut-off date, whichever occur first.

During post-treatment follow up:
Patients who discontinue study treatment due to PD: follow-up visit will be done every
3 months from the date of last study treatment administration until death or study cut-off date. Patients who discontinue the study treatment without PD: will be followed every month for disease assessment until confirmation of PD or start treatment with another anti-cancer therapy whichever comes first. After PD patient will be followed every 3 months as described just above until death or study cut-off date.

### Determination of end of clinical trial (all patients)

The cutoff date for primary analysis of ORR and reporting of other study endpoints will be 6 months after last patient first treatment. The final analysis cutoff date for OS analysis and updated analysis of ORR and other secondary endpoints will be approximately 12 months after last patient first treatment date.

While there have been shown and described what are at present considered the preferred embodiments of the invention, those skilled in the art may make various changes and modifications which remain within the scope of the appended claims.

## Claims

1. Isatuximab for use in combination with an anti-PD1 antibody for the treatment of multiple myeloma, wherein isatuximab is administered to a patient having previously received at least two prior lines of therapy, and wherein one of the prior lines of therapy is a proteasome inhibitor and one of the prior lines of therapy is an immunomodulatory agent.

2. Isatuximab for use according to claim 1, wherein said patient has previously received another anti-CD38 antibody.

3. Isatuximab for use according to claim 1 or 2, wherein isatuximab is administered at 10 mg/kg once every week for one 28 day cycle followed by 10 mg/kg every other week, and wherein the anti-PD1 antibody is administered at 1 to 3 mg/kg every other week.

4. Isatuximab for use according to claim 3, wherein the anti-PD1 antibody is administered at 3 mg/kg every other week.

5. Isatuximab for use according to claim 1 or 2, wherein isatuximab is administered at 10 mg/kg every other week, and the anti-PD1 antibody is administered at 1 mg/kg of every other week.

6. Isatuximab for use according to any one of claims 1 to 5, wherein the patient has received at least two months of treatment with the proteasome inhibitor and two months of treatment with the immunomodulatory agent prior to administering the isatuximab and the anti-PD1 antibody.

7. Isatuximab for use according to any one of claims 1 to 5, wherein the patient has received at least two cycles of treatment with the proteasome inhibitor and two cycles of treatment with the immunomodulatory agent prior to administering the isatuximab and the anti-PD1 antibody.

8. Isatuximab for use according to any one of claims 1 to 7, wherein the patient has
a) received three or more prior lines of therapy,
b) multiple myeloma that is refractory to a proteasome inhibitor,
c) multiple myeloma that is refractory to an immunomodulatory agent,
d) multiple myeloma that is refractory to a proteasome inhibitor and to an immunomodulatory agent,
e) relapsed multiple myeloma, and/or
f) relapsed and refractory multiple myeloma (RRMM).

9. Isatuximab for use according to claim 8, wherein the patient has multiple myeloma that is refractory to the most recent prior line of therapy.

10. Isatuximab for use according to any one of claims 1 to 9, wherein the most recent prior therapy received by the patient is another anti-CD38 antibody.

11. Isatuximab for use according to claim 2 or 10, wherein the other anti-CD38 antibody was daratumumab.

12. Isatuximab for use according to any one of claims 1 to 11, wherein said use does not include administration of dexamethasone.

13. Isatuximab for use according to any one of claims 1 to 12, wherein:
i. isatuximab and the anti-PD1 antibody are administered simultaneously; or
ii. isatuximab and the anti-PD1 antibody are administered sequentially; or
iii. isatuximab is administered before the anti-PD1 antibody.

14. Isatuximab for use according to any one of claims 1 to 13, wherein the anti-PD1 antibody comprises a heavy chain comprising the amino acid sequence of SEQ ID NO: 1 and a light chain comprising the amino acid sequence of SEQ ID NO: 6.

15. Isatuximab for use according to any one of claims 1 to 14, wherein the patient has at least one high risk cytogenic abnormality selected from the group consisting of del(17), t(4:14), and t(14:16).
